# EUROPEAN PATENT APPLICATION

(11) **EP 1 111 063 A1**
(43) Date of publication of application: **27.06.2001**
(21) Application number: 99204520.3
(22) Date of filing: 24.12.1999
(51) Int. Cl.: C12N 15/82, C12N 9/88, C12N 5/04, A23K 1/16, A01H 5/00

(54) **Modified DHPS genes**

(71) Applicant: Coöperatieve Verkoop- en Productievereniging van Aardappelmeel en Derivaten 'AVEBE' B.V., NL-9641 JA Veendam (NL)
(72) Inventor: Vorst, Oscar Frederick Jozef, 3572 HD Utrecht (NL); van der Meer, Ingrid Maria, 6703 AV Wageningen (NL); de Vetten, Nicolaas Clemens Maria Henricus, 9713 DE Groningen (NL)
(74) Representative: Ottevangers, Sietse Ulbe

(57) **Abstract**

The invention relates to modifications of biosynthesis key enzymes leading to essential amino acid accumulation in cells. A recombinant or isolated nucleic acid, or functional fragment thereof, encoding an enzyme dihydrodipicolinate synthase (DHPS), or functional fragment thereof, having been provided with a mutation leading to the replacement of at least one single amino acid residue with a cysteine residue.

### Annex to the application documents - subsequently filed sequences listing

SEQUENCE LISTING

<110> Coöperatieve Verkoop- en Productievereniging van Aardappelmeel
en
      Derivaten AVEBE B.V.

<120> Modified DHPS genes

<130> P51811EP00

<140> 99204520.3
<141> 1999-12-24

<160> 4

<170> PatentIn Ver. 2.1

<210> 1
<211> 1190
<212> DNA
<213> Solanum tuberosum

<220>
<223> Description of Combined DNA/RNA Molecule: CDS of
      dihydrodipicolinate synthase (DHPS)

<220>
<221> CDS
<222> (14)..(1075)

<400> 1
   ccgataggga gaa atg tca tcc tca att aca aat tgc tgc cat ttc tat     49
                  Met Ser Ser Ser Ile Thr Asn Cys Cys His Phe Tyr
                    1               5                  10
   gtt gaa cgt gcc aga aca aac agg aga act gca aca tgg aag tca cca     97
   Val Glu Arg Ala Arg Thr Asn Arg Arg Thr Ala Thr Trp Lys Ser Pro
            15                  20                  25
   aga gca gca gta att cca tgt atc cac ctc ccg atg cgc agt aat gaa     145
   Arg Ala Ala Val Ile Pro Cys Ile His Leu Pro Met Arg Ser Asn Glu
        30                  35                  40
   gtt aaa aat agg aca ttt gct gag gac ata aaa aaa ctt cga ttg att     193
   Val Lys Asn Arg Thr Phe Ala Glu Asp Ile Lys Lys Leu Arg Leu Ile
    45                  50                  55                  60
   act gca att aaa acc cca tat cta cca gat gga aga ttc gat ctt gag     241
   Thr Ala Ile Lys Thr Pro Tyr Leu Pro Asp Gly Arg Phe Asp Leu Glu
                    65                  70                  75
   gct tat gat gcc tta gta aat ttg caa att gaa aat ggt gtc gag ggt     289
   Ala Tyr Asp Ala Leu Val Asn Leu Gln Ile Glu Asn Gly Val Glu Gly
                80                  85                  90
   gtg att gtt ggt ggc act aca ggt gaa ggt caa ttg atg agc tgg gat     337
   Val Ile Val Gly Gly Thr Thr Gly Glu Gly Gln Leu Met Ser Trp Asp
            95                 100                 105
   gaa cac atc atg ctc att ggt cat acc atc aat tgt ttt ggt gga tca     385
   Glu His Ile Met Leu Ile Gly His Thr Ile Asn Cys Phe Gly Gly Ser
       110                 115                 120
   att aaa gtc att gga aac aca gga agc aac tcc acg agg gaa gca atc     433
   Ile Lys Val Ile Gly Asn Thr Gly Ser Asn Ser Thr Arg Glu Ala Ile
   125                 130                 135                 140
   cat gcg aca gaa caa gga ttt gct gta ggt atg cat gca gct ctt cac     481
   His Ala Thr Glu Gln Gly Phe Ala Val Gly Met His Ala Ala Leu His
                   145                 150                 155
   att aat cct tac tat ggg aag acc tcc ata gag ggt ttg att tct cac     529
   Ile Asn Pro Tyr Tyr Gly Lys Thr Ser Ile Glu Gly Leu Ile Ser His
               160                 165                 170
   ttt gac agt gtg ctc cct atg ggc cct act atc att tac aat gtg cca     577
   Phe Asp Ser Val Leu Pro Met Gly Pro Thr Ile Ile Tyr Asn Val Pro
           175                 180                 185
   tca cga act ggt caa gac att ccc cct cat gta atc cag aca tta gca     625
   Ser Arg Thr Gly Gln Asp Ile Pro Pro His Val Ile Gln Thr Leu Ala
       190                 195                 200
   aag agc cct aac ctt gct ggt gtc aaa gag tgt gta gga aat gat aga     673
   Lys Ser Pro Asn Leu Ala Gly Val Lys Glu Cys Val Gly Asn Asp Arg
   205                 210                 215                 220
   gtg gag cag tat aca agt aat ggg ttg gtt gtg tgg agt ggg aat gat     721
   Val Glu Gln Tyr Thr Ser Asn Gly Leu Val Val Trp Ser Gly Asn Asp
                   225                 230                 235
   gat gaa tgc cat gac tca agg tgg cat cat ggt gct acg gga gtt att     769
   Asp Glu Cys His Asp Ser Arg Trp His His Gly Ala Thr Gly Val Ile
               240                 245                 250
   tct gtc acc agc aac ttg gtt cct ggt ctg atg cgt gaa cta atg ttt     817
   Ser Val Thr Ser Asn Leu Val Pro Gly Leu Met Arg Glu Leu Met Phe
           255                 260                 265
   gga gga aat aac cct gtt ttg aac tca aag cta atg ccc ttg gtt gaa     865
   Gly Gly Asn Asn Pro Val Leu Asn Ser Lys Leu Met Pro Leu Val Glu
       270                 275                 280
   tgg ttg ttt cat gag cca aac ccc att gct ctg aat aca gct tta gct     913
   Trp Leu Phe His Glu Pro Asn Pro Ile Ala Leu Asn Thr Ala Leu Ala
   285                 290                 295                 300
   caa ctt ggg gtt gtg agg cct gtt ttt cgc ctt ccc tat gtt cca ctt     961
   Gln Leu Gly Val Val Arg Pro Val Phe Arg Leu Pro Tyr Val Pro Leu
                   305                 310                 315
   aca aag gca aag aga gag gag ttt gtg aag att gtt gag aaa att gga
   1009
   Thr Lys Ala Lys Arg Glu Glu Phe Val Lys Ile Val Glu Lys Ile Gly
               320                 325                 330
   cag gaa aac ttt att ggg gaa aga gat gtc caa gtt ttg gat gat att
   1057
   Gln Glu Asn Phe Ile Gly Glu Arg Asp Val Gln Val Leu Asp Asp Ile
           335                 340                 345
   gat ttt ttt ttt ggt tgg taggtattag tttttcccca attggtattg
   1105
   Asp Phe Phe Phe Gly Trp
       350
   agaagtaagt ttttgttgtt agcggcttaa tttgaaaaat ttcttgacat gaagatatct
   1165
   tctttatgtt gttttttaaa aaaaa
   1190

<210> 2
<211> 354
<212> PRT
<213> Solanum tuberosum
<223> Description of Combined DNA/RNA Molecule: CDS of
      dihydrodipicolinate synthase (DHPS)

<400> 2
Met Ser Ser Ser Ile Thr Asn Cys Cys His Phe Tyr Val Glu Arg Ala
1               5                   10                  15

Arg Thr Asn Arg Arg Thr Ala Thr Trp Lys Ser Pro Arg Ala Ala Val
            20                  25                  30

Ile Pro Cys Ile His Leu Pro Met Arg Ser Asn Glu Val Lys Asn Arg
        35                  40                  45

Thr Phe Ala Glu Asp Ile Lys Lys Leu Arg Leu Ile Thr Ala Ile Lys
    50                  55                  60

Thr Pro Tyr Leu Pro Asp Gly Arg Phe Asp Leu Glu Ala Tyr Asp Ala
65                  70                  75                  80

Leu Val Asn Leu Gln Ile Glu Asn Gly Val Glu Gly Val Ile Val Gly
                85                  90                  95

Gly Thr Thr Gly Glu Gly Gln Leu Met Ser Trp Asp Glu His Ile Met
            100                 105                 110

Leu Ile Gly His Thr Ile Asn Cys Phe Gly Gly Ser Ile Lys Val Ile
        115                 120                 125

Gly Asn Thr Gly Ser Asn Ser Thr Arg Glu Ala Ile His Ala Thr Glu
    130                 135                 140

Gln Gly Phe Ala Val Gly Met His Ala Ala Leu His Ile Asn Pro Tyr
145                 150                 155                 160

Tyr Gly Lys Thr Ser Ile Glu Gly Leu Ile Ser His Phe Asp Ser Val
                165                 170                 175

Leu Pro Met Gly Pro Thr Ile Ile Tyr Asn Val Pro Ser Arg Thr Gly
            180                 185                 190

Gln Asp Ile Pro Pro His Val Ile Gln Thr Leu Ala Lys Ser Pro Asn
        195                 200                 205

Leu Ala Gly Val Lys Glu Cys Val Gly Asn Asp Arg Val Glu Gln Tyr
    210                 215                 220

Thr Ser Asn Gly Leu Val Val Trp Ser Gly Asn Asp Asp Glu Cys His
225                 230                 235                 240

Asp Ser Arg Trp His His Gly Ala Thr Gly Val Ile Ser Val Thr Ser
                245                 250                 255

Asn Leu Val Pro Gly Leu Met Arg Glu Leu Met Phe Gly Gly Asn Asn
            260                 265                 270

Pro Val Leu Asn Ser Lys Leu Met Pro Leu Val Glu Trp Leu Phe His
        275                 280                 285

Glu Pro Asn Pro Ile Ala Leu Asn Thr Ala Leu Ala Gln Leu Gly Val
    290                 295                 300

Val Arg Pro Val Phe Arg Leu Pro Tyr Val Pro Leu Thr Lys Ala Lys
305                 310                 315                 320

Arg Glu Glu Phe Val Lys Ile Val Glu Lys Ile Gly Gln Glu Asn Phe
                325                 330                 335

Ile Gly Glu Arg Asp Val Gln Val Leu Asp Asp Ile Asp Phe Phe Phe
            340                 345                 350

Gly Trp

<210> 3
<211> 50
<212> DNA
<213> Artificial Sequence

<220>
<223> Description of Combined DNA/RNA Molecule: mutated DHPS

<220>
<223> Description of Artificial Sequence: mutated DHPS

<220>
<221> CDS
<222> (2)..(49)

<400> 3
a gtc att gga aac aca gga agc tgt tcc acg agg gaa gca atc cat gcg 49
  Val Ile Gly Asn Thr Gly Ser Cys Ser Thr Arg Glu Ala Ile His Ala
    1               5                   10                  15

a                                                                 50

<210> 4
<211> 16
<212> PRT
<213> Artificial Sequence
<223> Description of Artificial Sequence: mutated DHPS

<400> 4
Val Ile Gly Asn Thr Gly Ser Cys Ser Thr Arg Glu Ala Ile His Ala
  1               5                   10                  15

## Description

The invention relates to modifications of biosynthesis key enzymes leading to essential amino acid accumulation in cells.

Human and monogastric animals cannot synthesise 10 out of 20 amino acids and therefore need to obtain these essential amino acids from their diet. The essential amino acids are lysine, leucine, isoleucine, valine, phenylalanine, methionine, threonine and tryptophan. Additionally, tyrosine and cysteine, although they are not strictly essential, must be considered as such, since they are synthesised only from essential amino acids: tyrosine from phenylalanine and cysteine from methionine. The diet (herein also called food respectively feed) of man and his livestock is largely based on plant material. Among the essential amino acids needed for animal and human nutrition some amino acids, such as lysine, are often only present in relatively low concentrations in crop plants. Therefore, synthetic amino acids are usually added as supplements to grain-based and other diets, in order to increase the nutritional value of the diet. Dietary proteins are not nutritionally equivalent, which correlates with the amino acid composition of the different proteins. Feeding a diet that provides an inadequate amount of one of the essential amino acids leads to negative nitrogen balance, since the normal catabolism of proteins continues, but new synthesis for replacement is limited by the relative lack of the essential amino acid. This occurs even when the total dietary intake of protein is apparently adequate. The extent to which a dietary protein can be used for the synthesis of tissue proteins is limited by the content of the essential amino acid that is present in the least amount relative to the requirement. This is the limiting amino acid of that protein.

Many crop plants contain especially low levels of lysine. Because of this, the lysine content of plants is an agronomically important trait and various attempts have been made in the past to increase the level of lysine in plants by classical breeding, mutant selection or by genetic modification.

Regulation of amino acid biosynthesis is mainly based on feedback inhibition of the key enzymes from the pathway by the amino acid end products. Problems encountered by increasing the essential amino acid content in plants are due to the feedback inhibition mechanism.

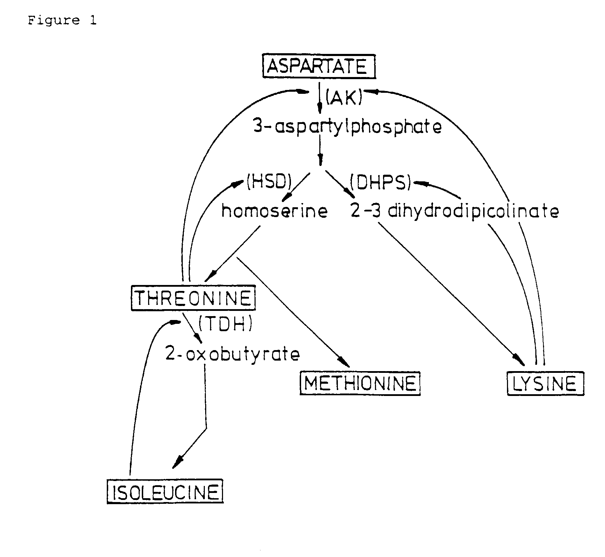

The essential amino acid lysine is synthesised, together with threonine and methionine, from aspartate by a complex pathway which is similar for bacteria and higher plants (Figure 1). The aspartate family pathway has been characterised in detail in *Escherichia coli* by isolation of enzymes involved in the pathway, which were later also purified from higher plants (Bryan, J.K. (1980), The Biochemistry of Plants (ed. by B.J. Miflin) Vol. 5: 403-452, Academic Press, N.Y.; Umbarger, H.E. (1978) Ann. Rev. Biochem. 47: 533-606).

The rate of synthesis of the aspartate-family amino acids is regulated primarily by a complex process of feedback inhibition of the activity of some key enzymes in the pathway by the relevant amino acid end product. The first enzymatic activity in the pathway that is common to all of the aspartate-family amino acids, aspartate kinase (AK) activity, is feedback inhibited by both lysine and threonine. In addition, lysine also inhibits the activity of the enzyme dihydrodipicolinate synthase (DHPS), the first enzyme of the pathway after the branch point that leads to the synthesis of lysine. AK catalyses the phosphorylation of aspartate to form 3-aspartyl phosphate, with the accompanying hydrolysis of ATP. Both in *E*. *coli* and in plants several different AK isoenzymes have been identified which are differentially inhibited either by lysine or by threonine. The product of AK activity, 3-aspartyl phosphate, is in the next enzymatic step converted to 3-aspartic semialdehyde (3-ASA), which serves as a common substrate for the synthesis of both lysine and threonine. The enzyme dihydrodipicolinate synthase (DHPS) catalyses the first reaction that is unique to lysine biosynthesis, the condensation of 3-aspartate semialdehyde with pyruvate to form 2,3-dihydrodipicolinate. In *E*. *coli* this enzyme is encoded by the dapA locus and appears to consist of four identical subunits as a homotetramer (Shedlarski, J.G. and Gilvarg, C. (1970) J.Biol.Chem. 245: 1362-1373). The *E*. *coli* dapA gene has been cloned and sequenced (Richaud, F. et al. (1986) J.Bacteriol. 166: 297-300). In plants DHPS enzyme also appears to be a single enzyme comparable to the *E*. *coli* enzyme. All plant DHPSs purified sofar were shown to possess activity that is strongly inhibited by lysine alone. Among the major regulatory enzymes of the aspartate family pathway in plants, DHPS is the most sensitive to feedback inhibition by its end product (I₅₀ of DHPS for lysine ranges between 10 and 50 microM). DHPS is about 10-fold more sensitive to lysine inhibition than are plant lysine-sensitive AKs (I₅₀ between 100 and 700 microM) and about 100-fold more sensitive to lysine inhibition than *E*. *coli* DHPS (I₅₀ is about 1mM) (Yugari, Y. and Gilvarg, C. (1962) Biochem.Biophys.Acta 62: 612-614; Galili, G. (1995) The Plant Cell 7: 899-906).

Several lines of evidence have indicated that in plants DHPS is the major rate-limiting enzyme for lysine synthesis. Mutants of several plant species possessing feedback-insensitive AK isozyme were found to overproduce free threonine, but exhibited only a slight increase in the level of lysine (Bright,S.W.J. et al. (1982) Nature 299: 278-279; Cattoir-Reynaerts A. et al. (1983) Biochem. Physiol. Pflanzen 178: 81-90; Dotson, S.B. et al. (1990) Planta 182: 546-552; Frankard, V. et al. (1992) Plant Physiol. 99: 1285-1293). On the other hand, a feedback-insensitive DHPS mutant tobacco plant overproduced lysine (Negrutiu, I. et al. (1984) Theor.Appl.Genet. 6: 11-20; Shaver, J.M. et al. (1996) P.N.A.S. USA 93: 1962-1966). Similar results have been reported with transgenic plants expressing feedback-insensitive DHPS or AK from *E*. *coli* (Glassman, K.F. (1992) Biosynthesis and Mol. Regul. of Amino Acids in Plants (ed. by B.K. Singh et al.): 217-228; Perl, A. et al. (1992) Plant Mol.Biol. 19: 815-823; Shaul, O. and Galili, G. (1992a) Plant J. 2: 203-209; Shaul, O. and Galili, G. (1992b) Plant Physiol. 100: 1157-1163). Transgenic plants that expressed the *E*. *coli* DHPS overproduced lysine, while those that expressed the *E. coli* AK overproduced threonine and exhibited only a slight increase in the level of lysine.

European Patent Application No. 429458 discloses a method of increasing the level of free L-lysine in a plant comprising (a) introducing a foreign gene into the cells of a plant tissue source and (b) expressing the foreign gene in the cells where a first DNA sequence of the gene encodes dihydrodipicolinate synthase (DHPS) which is resistant to feedback inhibition by endogenously produced free L-lysine. The foreign gene may comprise a second DNA sequence attached to the 5' terminus of the first DNA sequence and which encodes a chloroplast transit peptide (CTP) which localises the DHPS in the chloroplast of the cells. The plants are said to produce elevated levels of free lysine. European Patent Application No. EP 93908395 describes two isolated DNA fragments comprising a fragment encoding AK insensitive to inhibition by lysine and a second fragment encoding DHPS which is at least 20-fold less sensitive to inhibition by lysine than plant DHPS. It is claimed that the lysine-insensitive AK causes a higher than normal threonine production and that the DHPS causes a higher than normal lysine production in transformed plants.

The same results were found when feedback-insensitive bacterial DHPS and AK enzymes encoded by the *Corynebacterium* dapA gene and a mutant *E*. *coli* lysC gene, respectively, were expressed together in transgenic canola and soybean seeds. Several hundred-fold increases in free lysine in transgenic seed was observed, whereas the accumulation of excess threonine that was seen in transgenic seed expressing feedback-insensitive AK alone was prevented by the coexpression of DHPS (Falco,S.C. et al. (1995), Bio/Technology 13: 577-582). U.S. Patent Application No. 5773691 relates to four chimeric genes, a first encoding a bacterial lysine-insensitive aspartate kinase (AK), which is operably linked to a plant chloroplast transit sequence, a second encoding a bacterial lysine-insensitive dihydropicolinate synthase (DHPS), which is operably linked to a plant chloroplast transit sequence, a third encoding a lysine-rich protein, and a fourth encoding a plant lysine ketoglutarate reductase, all operably linked to plant specific regulatory sequences. The seeds of the transgenic plants are said to accumulate lysine or threonine to higher levels than untransformed plants.

The invention provides a recombinant or isolated nucleic acid, or functional fragment thereof, encoding an enzyme dihydrodipicolinate synthase (DHPS), or functional fragment thereof, having been provided with a mutation leading to the replacement of at least one single amino acid residue with a cysteine residue. The presence of an additional cysteine residue for example may result in the formation of alternative or additional sulphur bridges. As a result the overall structure and/or flexibilty of the enzyme will be affected, leading in the preferred cases to the loss of lysine feedback inhibition. In the present application a DHPS gene was used which was desensitised to lysine by at least one directed amino acid residue modification, preferably at the site of a in a wild-type enzyme conserved asparagine residue, and re-inserted into the cell of origin. In a preferred embodiment of the invention the replacement is located at or about the putative lysine binding site of DHPS, said site in *Escherichia coli* located at around position 75-85, preferably at the site of a in a wild-type enzyme conserved asparagine residue.

The mutated gene is preferably put back in its original environment to which its expression, activity and interaction with other components is maximally adapted. Changing the endogenous enzyme by changing at least a single amino acid residue in the enzyme in its original host, such as a yeast, fungus, a plant or a bacterium, will also be far more acceptable to public and consumers than replacing the gene by a heterologous one.

In particular, the invention provides mutation of plant DHPS genes; genes encoding DHPS from various plant species have already been cloned, such as from *Nicotiana sylvestris* (Ghislain, M. et al. (1995) Plant J.8: 733-743), soybean (Silk, G.W. (1994) Plant Mol Biol 26: 989-993), wheat (Kaneko, T. et al. (1990) J. of Biol. Chem. 265: 17451-17455) and maize (Frisch, D.A. et al. (1991) Mol. Gen. Genet. 288: 287-293). Plant DHPS mutants with reduced sensitivity to lysine have been sought in order to use the mutated plant genes for increase of free lysine in plants. Direct selection of tobacco (*Nicotiana sylvestris*) UV-irradiated tissue cultures for selection to the lysine analog S-2-aminoethyl-L-cysteine (AEC) resulted in a dominant mutation that reduced lysine inhibition of DHPS and increased the concentration of free lysine in leaves and seed (Negrutiu, I. et al. (1984) Theor. Appl. Genet. 68: 11-20). However, no other plants with altered DHPS inhibition have been obtained by AEC selection. The tobacco mutated DHPS gene showed to contain a dinucleotide mutation located in a 10-amino acid-long region that presumably identifies the lysine-binding site of DHPS enzyme (Ghislain, M. et al. (1995) Plant J. 8: 733-743). The mutation resulted in the change of a conserved asparagine amino acid residue into a isoleucine residue. The change to obtain a di-nucleotide mutation needed for the conversion of asparagine into isoleucine by UV irradiation is extremely low, which could explain the fact that no other AEC resistant plants could be rescued.

Two other groups tried to mutate the conserved region involved in lysine binding of the plant DHPS enzymes in different ways. Shaver et al. ((1996) Proc. Natl. Acad. Sci. 93: 1962-1966) expressed the maize DHPS cDNA in an *E. coli dapA* auxotroph, treated the cells with the mutagens ethylmethanesulfonate (EMS) and selected the cells on the lysine analogue AEC. They found several single nucleotide changes resulting in amino acid replacements causing nearly complete lack of lysine inhibition. However, no mutation was observed at the conserved asparagine residue which is changed in the potato DHPS enzyme as provided herein. Introduction of gene constructs containing these mutated maize DHPS genes into maize cell cultures resulted into an increase of four times the lysine content in transformed cells compared to wild type cells in the case of mutant DHPS166av (Bittel, D.C. et al. (1996) Theor. Appl. Genet. 92: 70-77). The use of this mutated maize DHPS gene is also mentioned in U.S. Patent Application No. 5545545.

The group of Silk and Matthews ((1997) Plant Mol Biol. 33: 931-933) used the information of the above mentioned mutations in tobacco and maize to change conserved amino acids in the soybean DHPS enzyme. The cloned soybean DHPS cDNA was site-directed mutated by PCR. One mutant contained a single amino acid substitution at codon 104 (conserved asparagine was changed into isoleucine at the same location in the tobacco DHPS mutant), in another mutant a single amino acid substitution was performed at position 112 (conserved alanine was changed into valine at the same location in the maize DHPS mutant). A third mutant contained both mutations. All mutants show the same lysine insensitivity when expressed in *E.coli dapA* auxotroph cells. There are at present no data on expression of these mutant DHPS gene constructs in plant cells.

When the amino acid sequences of several bacterial and plant DHPS enzymes are compared, a number of conserved amino acids become clear (Shaver et al. ((1996) Proc. Natl. Acad. Sci. 93: 1962-1966). The invariant lysine residue at position 161 in E.coli is required for binding pyruvate. Furthermore, there are three amino acid residues very conserved in the 10 amino acid long stretch of the DHPS enzyme which is probably or putatively involved in the binding of lysine. The site comprises the conserved glycine at position 78 (position in E. coli), a conserved asparagine at position 80, and a conserved threonine at position 82. An amino acid residue which according to the invention is preferably replaced by cysteine is the conserved asparagine at position 80 (position 134 in potato DHPS and position 158 in corn). This same conserved amino acid residue was mutated in a tobacco DHPS mutant, however it was changed into an isoleucine in stead of a cysteine. The mutation of asparagine, or of an amino acid close to that asparagine, in cysteine is apparently central to the increased lysine production of the transgenic organism, surprisingly, however, until now in none of cloned wild type and mutant bacterial, fungal or plant DHPS genes a cysteine residue is found at or around the (in wild type enzymes) conserved asparagine position.

The invention also provides a method for selecting a transformed host cell comprising a recombinant nucleic acid, or functional fragment thereof, encoding an enzyme dihydrodipicolinate synthase (DHPS), or functional fragment thereof, comprising culturing host cells in the presence of S-(2-aminoethyl)-L-cysteine and selecting a desired host cell for relative feedback insensitivity to S-(2-aminoethyl)-L-cysteine. Due to the combination of a randomised site-directed mutation method and selection for feedback insensitivity the invention provides an extremely insensitive DHPS mutant which, when for example expressed in potato plants, resulted in an accumulation of the free lysine content of more than 30% of the total free amino acid level in tubers. Increase of the lysine levels in potato is far more difficult than in, for instance tobacco, as can be seen by the experiments from Perl et al ((1993) Plant Mol Biol 19: 815-823) where expression of the bacterial DHPS (DapA) gene in potato resulted in far less lysine accumulation in potato than compared to expression of this gene in tobacco.

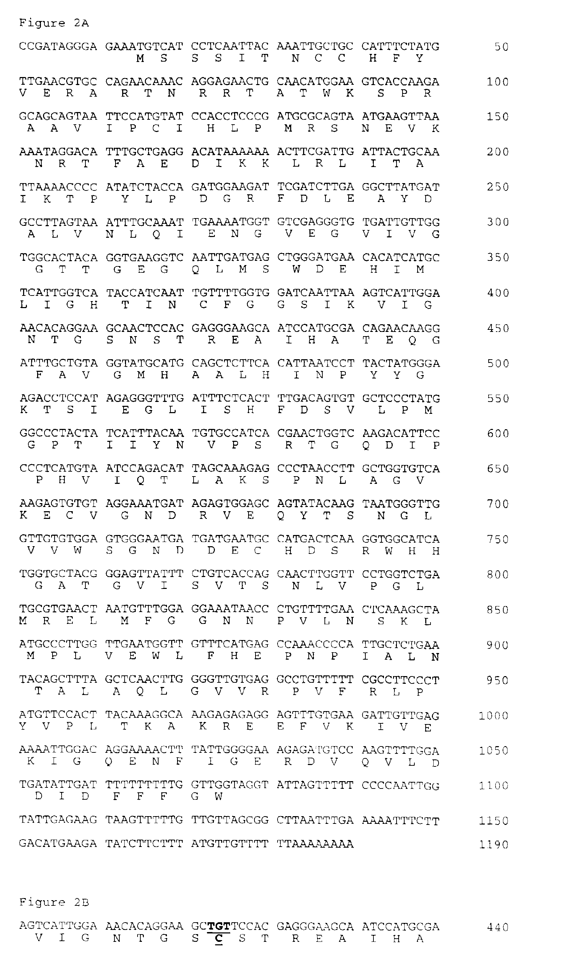

The invention also provides recombinant or isolated nucleic acid, or functional fragment thereof, encoding potato DHPS, or functional fragment thereof, preferably wherein said nucleic acid is at least 60%, preferably at least 75%, most preferably at least 90% homologous to a nucleic acid as shown in figure 2A. We mutagenised by PCR very specifically the conserved aspargine residue at position 134 in said potato DHPS enzyme into all other possible amino acid residues. The pool of mutant DHPS clones obtained in this way, containing all possible amino acid residues at position 134 was subsequently expressed in *E.coli dapA* auxotroph cells and selected for lysine insensitivity with the lysine analogue AEC. The conserved amino acid residue that had to be mutated was selected by us, but the change to another amino acid residue was driven by the selection in *E.coli dapA* cells.

The invention also provides a promotor or functional fragment thereof to drive expression of a DHPS gene as provided by the invention. Suitable promotors, such as plant-specific promotors are known in the art. In particular, the invention provides a nucleic acid according to the invention further comprising a tissue-specific promotor or functional fragment thereof. A preferred promoter to drive expression of the DHPS enzyme according to the invention is the tuber-specific granule bound starch synthase (GBSS) promoter (Visser, R.G., Plant. Mol. Biol. 17:691-699, 1991). Other tuber-specific promoters can be used to drive DHPS expression like class I patatin promoter from *Solanum tuberosum* (Mignery, C.A. et al. (1988) Gene 62: 27-44; Wenzler, H.C. et al. (1989) Plant Mol.Biol. 12: 41-50), AGPase promoter (Muller-Rober, B., et al., Plant Cell 6:601-612, 1994), proteinase inhibitor II promoter from potato (Keil, M. et al. (1989) EMBO J. 8: 1323-1330), or the cathepsin D inhibitor promoter from potato (Herbers, K. et al. (1994) Plant Mol. Biol. 26: 73-83). Also other tissue-specific promoters can be used like the fruit-specific promoter from tomato polygalacturonidase gene (Grierson, D. et al. (1986) Nucl.Acids Res. 14: 8595-8603), or the seed-specific phaseoline promoter from bean (Sengupta-Gopalan, C. (1985) Proc.Natl.Acad.Sci.USA 82: 3320-3324). Other promoters that can be used are inducible promoters, like the light inducible promoter derived from the pea rbcS gene (Coruzzi G. et al. (1984) EMBO J. 3: 1671-1679) and the actine promoter from rice (McElroy, D. et al. (1990) The Plant Cell 2: 163-171).

The promoter is in general to be found in the 5' region of each of the gene. Since the organelle in which lysine biosynthesis takes place in higher plants is the plastid, the gene construct comprises also a DNA sequence coding for a transit peptide which is involved in the translocation of the protein from the cytosol into the plastids (Van den Broeck, G. et al. (1985) Nature 313: 358-363; Schreier, P.H. et al. (1985) EMBO J. 4: 25-32). The chloroplast targeting signal can either naturally be present in a DHPS gene originating from a plant or has to be added when the DHPS gene originates from bacterial, fungal or algal organisms. This DNA sequence encoding a chloroplast transit peptide fused to a DNA sequence coding for DHPS, will on expression produce a fused DHPS/transit peptide chimeric protein in the cytoplasm of the transformed plant cell, which will be transported to the plastids, where increased production of lysine is thereby obtained.

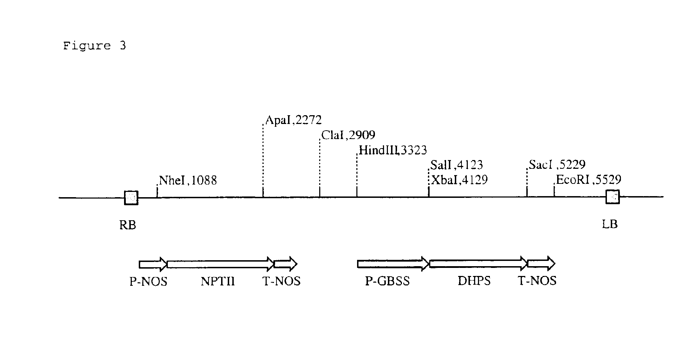

In a particular embodiment of the invention, the 3' end of the DNA sequence coding for the transit peptide is fused to the DNA sequence encoding DHPS, which is fused to a transcription termination DNA signal. This termination signal comprises a 3' transcription termination and a mRNA polyadenylation signal. Termination signals present at the 3' flanking region of any cloned gene can be used, e.g. from the pea rbcS gene, the bean phaseoline gene, or the nopaline synthase gene derived from the Ti plasmid of *Agrobacterium tumefaciens.* A preferred terminator sequence originates from the 3' flanking region of the octopine synthase gene from the Ti plasmid of *Agrobacterium tumefaciens* (Figure 3; Greve, H.D. et al. (1983) J.Mol.Appl.Genet. 1: 499-511).

The invention also provides a vector comprising a nucleic acid according to the invention. For example, an expression vector can be of the class of high copy number pUC or pBR322 derived plasmids, which can be used for direct transformation methods, like electroporation, particle bombardment or polyethylene-glycol mediated transformation procedures. Alternatively, expression constructs are included on a Ti plasmid derived, so-called binary vector, like pBINPLUS (Van Engelen, F.A. et al. (1995) Transgenic Research 4: 288-290). The Ti plasmids can be propagated in *Agrobacterium tumefaciens,* from which the inserted DNA fragments between the left and right border are transferred to the plant cell.

The expression vector in which the gene construct as provided by the invention, such as a chimeric gene construct, is cloned, is introduced into host cells such as fungal, bacterial, algal or plant cells. The introduction is realised using any kind of transformation protocol capable of transferring DNA; for plants several protocols have been developed for to either monocotyledonous or dicotyledonous plant cells. For example: transformation of plant cells by direct DNA transfer via electroporation (Dekeyser, R.A. et al. (1990) The Plant Cell 2: 591-602), via PEG precipitation (Hayashimoto, A. et al. (1990) Plant Physiol. 93: 857-863) or via particle bombardment (Gordon-Kann, W.J. et al. (1990) The Plant Cell 2: 603-618), and DNA transfer to plant host cells via infection with Agrobacterium. A preferred method is via infection of plant cells with *Agrobacterium tumefaciens* (Horsch, R.B. et al. (1985) Science 227: 1229-1231; Visser, R.G.F. (1991) Plant Tissue Culture Manual B5 (ed. by K. Lindsey): 1-9, Kluwer Acad. Publishers, The Netherlands). The methodology used here for in the detailed description for potato or grass is also provided to improve many other crop plants such as for example transgenic sugar beet, carrot, cassave, canola, alfalfa, legumes, and gramineae species like rice, maize, wheat, sorghum, barley and grasses like *Lolium perenne,* in a preferred embodiment of the invention, said plant comprises a tuber, such as a potato, from which lysine rich plant material can be obtained by tissue specific expression as provided herein. Other specific tissues wherein the DHPS-gene preferentially can be expressed comprise roots and seeds.

Transformed plants are for example selected by resistance to kanamycin or other antibiotics like hygromycin, or herbicides like bialaphos or phosphinotricin. Alternatively, desired transgenic plants or transgenic host cells (from which plants can be obtained) with a high lysine content are selected by cultivation in the presence of a lysine analogue, such as S-(2-aminoethyl)-L-cysteine (AEC), without selection on resistance to antibiotics or herbicides, as described above where E. coli host cells were selected. In a preferred example, the tissue-specific promoter that is used to drive DHPS expression is activated during the AEC selection. For example, the tuber-specific granule bound starch synthase (GBSS) promoter is induced by high levels of sucrose [Visser, R.G.F. et al. (1991) Plant Mol. Biol. 17:691-699), as is the AGPase promoter (Muller-Rober, B. et al. (1990) Mol. Gen. Genet. 224: 136-146) and the class I patatine promoter (Wenzler, H.C. et al. (1989) Plant Mol. Biol. 12: 41-50). Other tissue-specific promoters like the seed-specific phaseoline promoter from bean and the zein promoter from maize are induced during AEC selection by addition of ABA (Muller, M. et al. (1997) Plant J. 12 (2): 281; Bustos, M.M. et al. (1998) Plant Mol. Biol. 37(20): 265). No marker genes except for the DHPS gene thus have to be introduced.

The invention furthermore provides a method to obtain plant material with relative high lysine content comprising harvesting at least a part of a plant according to the invention. Harvesting crop plants and obtain specific plant material, such as seeds, leaves, stems, roots, tubers or fruits, is a skill known in the art, allowing to obtain plant material from a plant according to the invention. The invention thus provides a method to increase lysine content of food or feed with relative low lysine content comprising adding plant material according to the invention to said food or feed. Lysine rich food or feed is also provided, and last but not least, it is in itself possible to use the lysine-enriched plant material according to the invention, such as lysine enriched potatoes, grasses, seeds or fruits as food or feed. The invention is further explained in the detailed description without limiting the invention.

### Detailed description.

### Example 1. Cloning of the potato DHPS cDNA

DNA isolation, subcloning, restriction analysis and DNA sequence analysis is performed using standard methods (Sambrook, J. et al. (1989) Molecular Cloning. A laboratory manual, Cold Spring Harbor Laboratory Press; Ausubel, F.M. et al. (1994) Current protocols in molecular biology, John Wiley & Sons).

For the isolation of the *Solanum tuberosum* cv Kardal gene encoding dihydrodipicolinate synthase (DHPS) a cDNA library of mRNA prepared from young tubers was constructed in the vector λTriplEx (Clotech). A specific 600 basepairs DHPS DNA fragment for screening the cDNA library via heterologous hybridisation was cloned by the reverse transcriptase polymerase chain reaction (RT-PCR) on total RNA isolated from *Arabidopsis thaliana* and subsequently ligated into vector pMOSBlue (Amersham), resulting in pAAP15. To this purpose synthetic oligonucleotides based on the sequence of the *Arabidopsis thaliana* DHPS gene were used (Vauterin & Jacobs, 1994).

A single plaque from the cDNA hybridising to pAAP15 was selected and the 1190 basepairs DNA insert completely sequenced (Figure 2A). It was concluded to encode a dihydridipicolinate synthase. The DNA fragment encompassing the complete coding sequence was cloned in the cloning vector pBluescript SK(+) (Stratagene) via a PCR-based strategy to enable the expression in *Escherichia coli* of a β-galactosidase-DHPS fusion protein under the control of a *lac* promoter. The resulting plasmid was designated pAAP57. This gene construct was able to complement *E. coli* AT997 (Yeh et al., 1988), a DHPS deficient strain, allowing it to grow on minimal medium in the absence of DL-α, ε-diaminopimelic acid.

### Example 2. Mutaqenesis to create a lysine insensitive DHPS gene

In order to create a feedback insensitive DHPS, nucleotides in pAAP57 corresponding to the evolutionary conserved amino acid residue 134 (asparagine) were changed at random via a PCR-based approach (QuickChange site directed mutagenesis, Stratagen), resulting in a population of plasmids encoding DHPS enzymes with different amino acids residues at position 134. Following transformation of *E*. *coli* AT997 with this plasmid population, selection for feedback insensitivity was done in the presence of 1 mM of the lysine analogue S-(2-aminoethyl) -L-cysteine (AEC). Several AEC resistant colonies were picked and the DHPS coding region of their plasmid directing AEC resistance sequenced (Figure 2B). The change of AAC into either TGT or TGC resulted in the corresponding change of the asparagine residue at DHPS position 134 into a cysteine residue. The mutant DHPS encoding DNA fragment (designated DHPS-134nc1) was used for the expression in potato plants.

### Example 3. Chimeric gene construct with the mutant potato DHPS gene

The chimeric gene containing the mutant DHPS gene was constructed by subcloning DHPS cDNA from the pTriplex vector (pAAP42) first in pCR-Script SK(+) (pAAP55) and from this vector as a XbaI-Eco RI fragment in the pBluescript SK vector digested with XbaI-EcoR (pAAP57). With this clone the mutagenesis was performed, resulting in clone pAAP57-134nc1. At the 5'end the mutated DHPS cDNA was fused to a HindIII-SalI fragment of the 800 bp long GBSS promoter fragment (Visser et al. ibid). Downstream of the mutant DHPS sequence the termination signal of the nopaline synthase gene from *Agrobacterium tumefaciens* was inserted (Greve, H.D. et al. (1983) J.Mol.Appl.Genet. 1: 499-511) as an SstI-EcoRI fragment. The complete chimeric gene was subcloned into the HindII-EcoRI sites of pBINPLUS (Van Engelen, F.A. et al. (1995) Transgenic Research 4: 288-290) (pAAP105, Figure 3).

### Example 4. Introduction of the chimeric gene into potato

### 4.1 Transformation of potato plants

The binary vector pAAP105 was used for freeze-thaw transformation of *Agrobacterium tumefaciens* strain AGLO (Höfgen, R. and Willmitzer, L. (1988) Nucl.Acids Res. 16: 9877). Transformed AGLO was subsequently used for inoculation of potato *(Solanum tuberosum,* variety Kardal) stem explants as described by Visser (Visser, R.G.F. (1991) Plant Tissue Culture Manual B5 (ed. by K. Lindsey): 1-9, Kluwer Acad. Publishers, The Netherlands). After shoot and root regeneration on kanamycin-containing media plants were put in soil and transferred to the greenhouse. Plants regenerated (on kanamycin-free media) from stem explants treated with the Agrobacterium strain AGLO lacking a binary vector served as a control.

### 4.2 Selection of transformed plant cells on AEC

In stead of selection of transformed plant cells on kanamycine, as described by Visser (Visser, R.G.F.(1991) in : K. Lindsey (ed) Plant Tissue Culture Manual B5: 1-9, Kluwer Acad Publishers) mentioned above (4.1), the transformed plant cells containing a mutant DHPS gene can also be selected on the lysine analogue AEC. After inoculation of for example potato stem explants with the transformed (AGI 0) strain, explants are in general first cultured on medium without AEC. After a short period, for example 12-20, such as 16, days they are transferred to medium with 0.1 mM AEC and a sufficient concentration (e.g. 5%) sucrose. When the first primordia are visible the explants are transferred to 0.025 mM AEC and sucrose. Root induction is performed in the absence of AEC/sucrose-selection.

### 4.3 In vitro tuber formation

In order to induce in vitro tuberisation, nodal cuttings (about 4-5 cm long) of transformed potato plantlets are placed vertically in solid Murashige and Skoog medium (Murashige, T. and Skoog, F. (1962) Physiol.Plant. 15: 473-497) supplemented with 10% (w/v) sucrose, 5 TM BAP. The cultures are maintained at 19°C in the dark. After 14 days microtubers of 4 mm in diameter are harvested and analysed for free lysine and threonine content, and for AK and DHPS activity. The protein content and the protein composition are analysed as described below.

### Example 5. DHPS modification of perennial ryegrass (Lolium perenne L.)

### Plant materials

Embryogenic suspension cultures from different *Lolium perenne* cultivars, a.o. the cultivars Moronda and Aurora, are initiated directly from mature, seed-derived embryos or from embryogenic callus cultures obtained from immature inflorescence segments, essentially as described by Creemers-Molenaar et al., *Plant Science* 63:167-176 (1989). For the direct approach, seeds are sterilized in 10% hypochlorite, rinsed and soaked for two days in sterile tap water and sterilized for a second time. After rinsing thoroughly, mature embryos are dissected from 40 seeds, chopped and transferred to 5 ml of MS10 medium (i.e. Murashige and Skoog basal salts and vitamins supplemented with 10 mg/l 2,4-D and 3% sucrose at pH 5.8) in a 60 ml plastic specimen container (Thovadex). This in several replicates. Embryogenic callus is induced on immature inflorescence segments of greenhouse grown plants after sterilization with 5% hypochlorite and rinsing with sterile water. The basal parts of the inflorescences are cut into 2 segments of 1-2 mm long and placed on MSt5 medium (i.e. MS basal salts and vitamins supplemented with 0.4 mg/l (=extra) thiamin-HCl, 5 mg/l 2,4-D and 3% sucrose) solidified with 0.8% Daichin agar. Culture is in the dark at 25 °C. After 4-8 weeks compact, embryogenic callus is excised from the explants of 2-5 plants (i.e. genotypes). The calli are mixed, chopped with a scalpel and transferred in 0.1 g FW aliquots to 5 ml of MS10 in specimen containers. From this point on, the cultures of both origins are treated identically. The cultures are incubated on a rotary shaker (140 revs./min.) in continuous indirect light (200-400 lux) at 25 °C.

After 10 days the medium is replaced with MS5 ( = as MS10 but with 5 mg/l 2,4-D). Once a week 2 ml of fresh medium is added to the cultures until a final volume of 15 ml is reached. Subsequently, the cultures are transferred to new 190 ml transparent, polystyrene containers (Greiner) and 5 ml fresh medium is added. For further experimentation well-proliferating, finely-dispersed cultures are selected and maintained by weekly subculturing 2.5 g FW material in 20 ml fresh MS5. Incubation is in the dark under continuous shaking at 120 revs./min. at 25 °C.

The regeneration potential of the cultures is determined by placing 0.5-1.0 g FW material on solid MS0 (0 mg/l 2,4-D; 0.8% agar). After culturing in the dark at 25 °C for the first 2 weeks the calli are placed in dimmed light (500-1000 lux) for 16 hours/day for the next 2 weeks. Finally after transfer to fresh MS0, they are placed in 4000 lux, 16 hours/day and the number of calli producing shoots is scored after 4 weeks.

### Transformation

Three days after subculture, 0.25 g FW callus material consisting of cells in log-phase, is evenly dispersed onto the surface of a 42 mm diameter Whatman filter disc (Schleicher & Schuell #604). Subsequently, the filters are moistened by the addition of 0.5 ml fresh culture medium and they are placed onto culture medium solidified with 0.2% gelrite and left overnight at 25 °C in the dark. The next day the filters carrying the perennial ryegrass suspension material are used for biolistic gene transfer using the PDS1000-He particle gun (BioRad). 0.375 mg Gold particles with an average diameter of 1 µm were coated with 0.625 µg DNA of plasmid pAAP205, which is a derivative of the plasmid pAAP105 mentioned in Example 3 lacking T-DNA borders and with the *npt*II gene replaced by the *hpt* gene for selection of transgenic perennial ryegrass cells. For the coating, 50 µl (= 3 mg) of washed particles are suspended thoroughly by vortexing. Subsequently, 5 µl plasmid DNA (concentration 1 µ g/µl) and 50 µl 2.5M CaCl₂ are added and vortexed for 10 seconds. Then, 20 µl 0.1M free-base spermidine is added and mixed by vortexing for 2 seconds. The mixture is centrifuged for 5 seconds and the supernatant is removed, after which 250 µl ethanol 96% is added followed by vortexing for 1 minute. After washing with ethanol once, the particles now coated with DNA are resuspended in 60 µl ethanol 96% and kept on ice until use. Bombardment can be performed at pressures ranging from 1100 psi to 2200 psi, but in this example particularly a pressure of 1800 psi is used while the dish containing the filters is placed at a distance of 9 cm. After biolistics the filters are incubated on the culture medium in the dark at 25 °C for 24 hours before they are transferred to selection medium. For selection, the filters are first placed on culture medium MSt5 solidified with 0.2% gelrite containing 80 mg/l hygromycin; after 1 week the filters are transferred to selection medium containing 150 mg/l hygromycin. So far, this is essentially as described earlier (Van der Maas et al., *Plant Mol. Biol.* 24:401-405 [1994]). Actively growing calli are individually transferred to fresh selection medium (150 Hyg.) after 4 weeks. Following this second round of selection surviving calli are placed on regeneration medium supplemented with 50 mg/l hygromycin. Transgenic perennial ryegrass plants are obtained and collected after 8 weeks. They are maintained in tubes containing half strength MS0 (Creemers-Molenaar et al., Plant *Science* 57: 165-172 [1988]) and subsequently characterized molecularly by PCR and Southern hybridization analysis and biochemically by amino acid, enzyme activity and protein analysis as described in Example 6 - 9 to confirm presence and expression of the newly introduced gene constructs.

### Example 6. Analysis of free amino acid content in transgenic plants

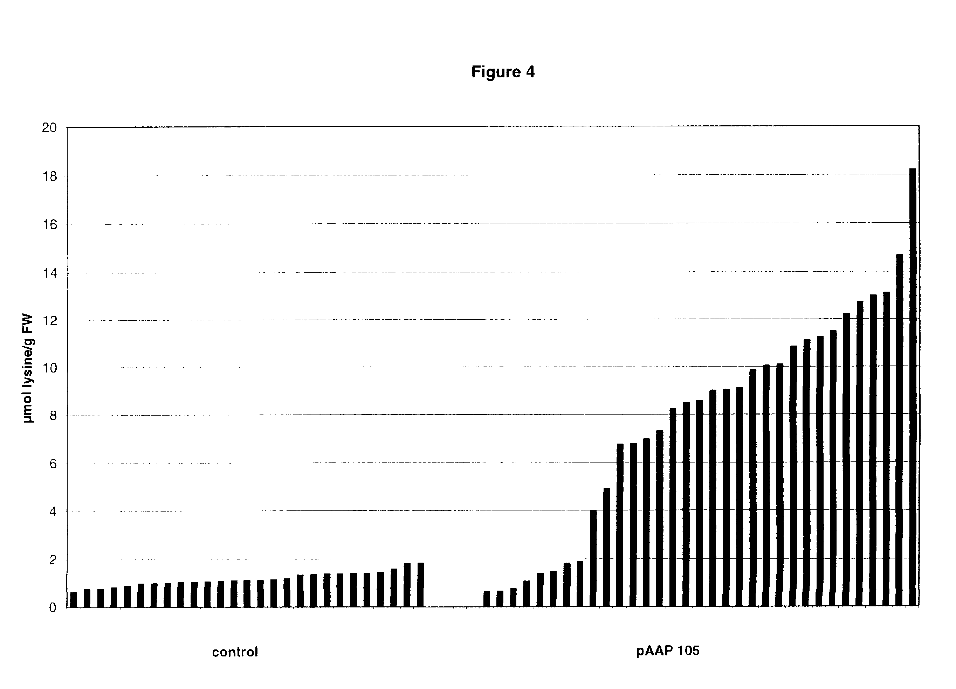

Tissue (0.5-1.0 gram) was homogenised with mortar and pestle in 2 ml 50 mM Pi-buffer (pH 7.0) containing 1 mM dithiothreitol. Nor-leucine is added as an internal standard. Free amino acids were partly purified by extraction with 5 ml of a water:chloroform:methanol mixture (3:5:12). Water phase was collected and the remaining re-extracted twice. After concentration by lyophilisation to 3 ml, a 20 µl sample was analysed by HPLC using a cation-exchange column with post-column ninhydrine derivatisation of the amino acids detected at 570 and 440 nm (BIOCHROM 20, Amersham Pharmacia biotech). Figure 4 shows the lysine levels as the percentage of the total amino acids in tubers in 27 untransformed potato plants and in 30 potato plants containing the mutated DHPS gene construct pAAP105. The lysine levels increase from 2-2.5% in the control wild type tubers to a maximum percentage of 30 in the transformed tubers, resulting in lysine being a 'bulk' amino acid in stead of a 'low level' essential amino acid .

### Example 7. Analysis of DHPS enzyme activity in transgenic plants

Microtubers or mature tubers were homogenised with mortar and pestle in an equal volume of cold 100mM Tris-HCl pH 7.5 containing 2 mM EDTA, 1.4% sodium ascorbate, 1mM phenylmethylsulphonilfluoride and 0.5 Tg/ml leupeptin. Following 5 min. centrifugation (16,000 g at 4°C) the supernatant is collected. DHPS activity was measured using the O-aminobenzaldehyde (O-ABA) method of Yugari and Gilvarg (Yugari, Y. and Gilvarg, C. (1965) J.Biol.Chem. 240: 4710-4716). As expected, AEC sensitivity of transformed was reduced, when comparing the wild type and mutant DHPS activity in respectively control (untransformed) and transformed potato tubers, the latter showed DHPS acitivity even under increased levels of AEC.

### Example 8. DNA analysis of transgenic plants

Genomic DNA was isolated from potato tubers of plants grown in the green house. Lyopholised tissue was ground in liquid nitrogen and added to 15 ml extraction buffer (100mM Tris.HCl, pH 8, 500mM NaCl, 50mM EDTA and 10mM ß-mercaptoethanol) and 2 ml 10% SDS. After incubation at 65°C for 20 min, 5 ml of 5M KAc were added and incubated on ice for 15 min. After centrifugation, filtration of the supernatant, and precipitation with 15 ml iso-propanol, the pellet was resuspended in 400 µl TE and treated with 1 µg RNAse. The DNA was subsequently purified with CTAB (N-Cetyl-NNN tri-methylammonium bromide) extraction by adding 400 µl of CTAB buffer (200mM Tris-HCl pH 7.5, 50mM EDTA, 2mM NaCl and 2% CTAB) and incubating at 65 0C for 15 min. After extraction with 800 µl chloroform/isoamyl alcohol 24:1, the DNA was precipitated with 800 µl iso-propanol. The pellet was washed with 70% ethanol and resuspended in TE. DNA. After digestion with restriction enzymes, the DNA was size-fractionated on an agarose gel and blotted onto Hybond-N⁺ (Amersham). Random-primer-labeled probe was derived from the DHPS cDNA (from position 337 to 811, Figure 2). Filters were hybridised at 65 °C in 10 % dextran sulphate, 1% SDS and 1M NaCL for 16 to 20 hr. and washed subsequently at 55 °C and 60 °C with 2x SSC, 0.1 % SDS, and at 65 °C with 0.1x SSC, 0.1% SDS.

### Example 9. Expression analysis of transgenic plants on RNA level

Total RNA was isolated from potato tubers grown in the greenhouse according to the protocol described by Ausubel et al. (1994). RNA was size-fractionated on formaldehyde agarose gels and blotted onto Hybond-N⁺ (Amersham). Blots were briefly stained to ensure that equal amounts of RNA were present. Random-primer-labeled probe was derived from the DHPS cDNA (from position 337 to 811, Figure 2). Filters were hybridised at 65 °C in 10 % dextran sulphate, 1% SDS and 1M NaCL for 16 to 20 hr. and washed subsequently at 55 °C and 60 °C with 2x SSC, 0.1 % SDS, and at 65 °C with 0.1x SSC, 0.1% SDS.

### Figure legends

fig.1:
   A diagram of the aspartate family biosynthetic pathway. Only the major key enzymes are indicated. Curved arrows represent feedback inhibition by the end product amino acids DHPS, dihydrodipicolinate synthase; HSD, homoserine dehydrogenasse; TDH, theonine dehydratase.
fig.2:
   (A) Nucleic acid fragment encoding a DHPS isolated from potato. The derived amino acid sequence is presented using the 1-letter code.
   (B) Nucleic acid sequence and derived amino acid sequence of a lysine binding domain of the mutated DHPS (DHPS-134nc.)
fig.3:
   Schematic representation of the T-DNA region of plasmid pAAP105, encompassing a mutated dihydrodipicolinate coding region (DHPS) under control of the granule bound starch synthase promoter (P-GBSS) and as selection maker the neomycinephosphotransferase 11 coding region (NPT11) under control of the nopaline synthase promoter(P-NOS) ;I-NOS nopaline synthase transcription terminator.
fig.4:
   Free lysine concentration(micromol/g fresh weight) of tubers from tubers derived from different, independent transformant or control plants. Plants were grown in pots in the green house until maturity. After harvesting tubers were dried for one week before analysis. About 10 gram of tuber material was ground in 50 ml of a 1 mM dithiothreitol (DTT) solution. Two ml of the resulting suspension was extracted with a menthanol: water: chloroform mixture(12:3:5). After concentration to 0.5 ml, 25µl is used for analysis on a Biochrom 20(Amersham-Phamacia) lithium-based ionexchange amino acid analysis system. Samples were spiked with L-Norleucine before grinding, to allow corrections.

### REFERENCES

- Ausubel, F.M. et al. (1994) Current protocols in molecular biology, John Wiley & Sons
- Benfey, P.N. et al. (1990) EMBO J. 9: 1685-1696.
- Black, S. et al. (1955) J.Biol.Chem. 213: 27-38.
- Brederode, F.T. et al. (1980) Nucl.Acids Res. 8: 2213-2223.
- Bright,S.W.J. et al. (1982) Nature 299: 278-279.
- Bryan, J.K. (1980), The Biochemistry of Plants (ed. by B.J. Miflin) Vol. 5: 403-452, Academic Press, N.Y.
- Casan, M. et al. (1986), J.Biol.Chem. 261: 1052-1057.
- Cattoir-Reynaerts A. et al. (1983) Biochem.Physiol.Pflanzen 178: 81-90.
- Cohen and De Antonis (1994) J. of Chromatography 661: 25-34.
- Coruzzi G. et al. (1984) EMBO J. 3: 1671-1679.
- Dekeyser, R.A. et al. (1990) The Plant Cell 2: 591-602
- Dotson, S.B. et al. (1990) Planta 182: 546-552.
- Dunn,M.J., Corbett,J.M. Meth. Enzymol. (1996) V271, 177-203.
- Falco,S.C. et al. (1995), Bio/Technology 13: 577-582.
- Fluhr, R. et al. (1986) EMBO J. 5: 2063-2071.
- Frankard, V. et al. (1992) Plant Physiol. 99: 1285-1293.
- Galili, G. (1995) The Plant Cell 7: 899-906.
- Gallie, D.R. et al. (1987) Nucl.Acids Res. 15: 3257-3273.
- Glassman, K.F. (1992) Biosynthesis and Mol. Regul. of
   Amino Acids in Plants (ed. by B.K. Singh et al.): 217-228.
- Görg, A. et.al, (1985) Electrophoresis V6,599-604.
- Gordon-Kann, W.J. et al. (1990) The Plant Cell 2: 603-618.
- Greve, H.D. et al. (1983) J.Mol.Appl.Genet. 1: 499-511.
- Grierson, D. et al. (1986) Nucl.Acids Res. 14: 8595-8603.
- Hayashimoto, A. et al. (1990) Plant Physiol. 93: 857-863.
- Herbers, K. et al. (1994) Plant Mol. Biol. 26: 73-83.
- Hötgen, R. and Willmitzer, L. (1988) Nucl.Acids Res. 16: 9877.
- Horsch, R.B. et al. (1985) Science 227: 1229-1231.
- Keil, M. et al. (1989) EMBO J. 8: 1323-1330).
- Laemmli, U.K. (1970) Nature V227, 680-685 Lea, P.J. et al. (1985) Chemistry and Biochemistry of the Amino Acids (ed. by G.C. Barrett): 197-226, London: Chapman and Hall.
- Manneberg, M et al. (1995) Anal. Biochem. 224: 122-127.
- Matthews, B.F. et al. (1989) Plant Physiol. 91: 1569-1574.
- McElroy, D. et al. (1990) The Plant Cell 2: 163-171.
- Mignery, C.A. et al. (1988) Gene 62: 27-44.
- Murashige, T. and Skoog, F. (1962) Physiol.Plant. 15: 473-497.
- Negrutiu, I. et al. (1984) Theor.Appl.Genet. 6: 11-20.
- Pen, J. et al. (1992) Bio/Techn. 10: 292-296.
- Perl, A. et al. (1992) Plant Mol.Biol. 19: 815-823.
- Richaud, C. et al.(1973) Eur.J.Biochem. 40: 619-629.
- Richaud, F. et al. (1986) J.Bacteriol. 166: 297-300.
- Sambrook, J. et al. (1989) Molecular Cloning. A laboratory manual, Cold Spring Harbor Laboratory Press.
- Schreier, P.H. et al. (1985) EMBO J. 4: 25-32.
- Sengupta-Gopalan, C. (1985) Proc.Natl.Acad.Sci.USA 82: 3320-3324.
- Shaul, O. and Galili, G. (1992a) Plant J. 2: 203-209.
- Shaul, O. and Galili, G. (1992b) Plant Physiol. 100: 1157-1163.
- Shaul, O. and Galili, G. (1993) Plant Mol. Biol. 23: 759-768.
- Shedlarski, J.G. and Gilvarg, C. (1970) J.Biol.Chem. 245: 1362-1373.
- Smeekens, S. et al. (1985a) Nucl.Acids Res. 13: 3179-3194.
- Smeekens, S. et al. (1985b) Nature 317: 456-458.
- Umbarger, H.E. (1978) Ann.Rev.Biochem. 47: 533-606.
- Vauterain, M. and Jacobs, M. (1994) Plant Mol. Biol. 14: 545-550.
- Van den Broeck, G. et al. (1985) Nature 313: 358-363.
- Van Engelen, F.A. et al. (1995) Transgenic Research 4: 288-290.
- Van Gelder,W.M.J. and Vonk,C.R. (1980) Potato Res. V23 427-434.
- Visser, R.G.F. (1991) Plant Tissue Culture Manual B5 (ed. by K. Lindsey): 1-9, Kluwer Acad. Publishers, The Netherlands.
- Wenzler, H.C. et al. (1989) Plant Mol.Biol. 12: 41-50.
- Yeh, P. et al. (1988) Mol Gen. Genet. 212:105-111.
- Yugari, Y. and Gilvarg, C. (1962) Biochem.Biophys.Acta 62: 612-614.
- Yugari, Y. and Gilvarg, C. (1965) J.Biol.Chem. 240: 4710-4716.

## Claims

1. A recombinant or isolated nucleic acid, or functional fragment thereof, encoding an enzyme dihydrodipicolinate synthase (DHPS), or functional fragment thereof, having been provided with a mutation leading to the replacement of at least one single amino acid residue with a cysteine residue.

2. A nucleic acid according to claim 1 wherein said amino acid replacement is located at or about the putative lysine binding site of DHPS, said site in *Escherichia coli* located at around position 75-85.

3. A nucleic acid according to claim 1 or 2 wherein said single amino acid residue comprises an asparagine residue.

4. A nucleic acid according to anyone of claims 1 to 3 wherein in potato DHPS said asparagine residue is located at position 134.

5. A nucleic acid according to anyone of claims 1 to 4 wherein said DHPS is of bacterial, fungal, algal or plant origin.

6. A nucleic acid according to claim 5 wherein said plant is a potato.

7. A recombinant or isolated nucleic acid, or functional fragment thereof, encoding potato DHPS, or functional fragment thereof.

8. A nucleic acid according to claim 7 wherein said nucleic acid is at least 90% homologous to a nucleic acid as shown in figure 2A.

9. A nucleic acid according to anyone of claims 1 to 8 further comprising a tissue-specific promotor or functional fragment thereof.

10. A nucleic acid according to claim 9 wherein said promotor is derived from the tuber-specific granule bound starch synthase (GBSS) promotor.

11. A vector comprising a nucleic acid according to anyone of claims 1 to 10.

12. A host cell comprising a nucleic acid according to anyone of claims 1 to 10 or a vector according to claim 11.

13. A host cell according to claim 12 comprising a plant cell.

14. A host cell according to claim 13 wherein said plant cell is derived from a potato.

15. A method for selecting a transformed host cell comprising a recombinant nucleic acid, or functional fragment thereof, encoding an enzyme dihydrodipicolinate synthase (DHPS), or functional fragment thereof, comprising culturing host cells in the presence of S-(2-aminoethyl)-L-cysteine and selecting a desired host cell for relative feedback insensitivity to S-(2-aminoethyl) -L-cysteine.

16. A method according to claim 15 wherein said nucleic acid comprises a nucleic acid according to anyone of claims 1 to 10.

17. A host cell obtainable with a method according to claim 15 or 16.

18. A plant comprising a host cell according to claim 14 or 17.

19. A plant according to claim 18 comprising a crop plant.

20. A plant according to claim 18 or 19 comprising a tuber.

21. A tuber derived from a plant according to claim 20.

22. A method to obtain plant material with relative high lysine content comprising harvesting at least a part of a plant according to claim 18.

23. Plant material obtainable by a method according to claim 22.

24. A method to increase lysine content of food or feed with relative low lysine content comprising adding plant material according to claim 23 to said food or feed.

25. Food or feed obtainable by a method according to claim 24.

26. Food or feed comprising plant material according to claim 24.
